(19) Europäisches Patentamt
European Patent Office
Office européen des brevets

(11) Publication number: **0 241 830 A2**

(12) **EUROPEAN PATENT APPLICATION**

(21) Application number: 87105018.3

(22) Date of filing: 04.04.87

(51) Int. Cl.4: C 07 K 15/00, C 07 K 7/10, C 07 K 3/28, A 61 K 39/395, A 61 K 37/36, G 01 N 33/53

(30) Priority: 08.04.86 US 849541

(43) Date of publication of application: 21.10.87 Bulletin 87/43

(84) Designated Contracting States: AT BE CH DE ES FR GB GR IT LI LU NL SE

(71) Applicant: THE GENERAL HOSPITAL CORPORATION, 55 Fruit Street, Boston MA 02114 (US)

(72) Inventor: Smith, John A., 19 Thatcher Street Apartment no. 5, Brookline Massachusetts 02146 (US)

(74) Representative: Meyer-Dulheuer, Karl-Hermann, Dr. et al, HOECHST Aktiengesellschaft Zentrale Patentabteilung Postfach 80 03 20, D-6230 Frankfurt/Main 80 (DE)

(54) **Hepatoma-derived growth factor.**

(57) This invention is directed to purified hepatoma-derived growth factor, which is an endothelial mitogen and a potent angiogenic factor, pharmaceutical composition comprising said factor and processes for preparing said factor.

EP 0 241 830 A2

**HEPATOMA-DERIVED GROWTH FACTOR**

Technical Field

This invention is directed to the purification to homogeneity of a tumor-derived endothelial cell growth factor from hepatoma cells (hepatoma-derived growth factor). The invention also concerns the uses of the hepatoma-derived growth factor in controlling angiogenesis and detecting cancerous liver tumors.

Background Art

Endothelial cell growth factors are diffusible protein mitogens which induce angiogenesis, the growth of new blood vessels. Maciag et al., Science 225:932-935 (1984). In normal tissue, the endothelial mitogenic factors are important for a variety of physiological functions, including wound repair. Angiogenesis of tumors occurs when a growth factor is released by tumor cells. The growth factor induces the growth of new blood vessels from surrounding tissue into the solid tumor. The proliferation of new blood vessels to the tumor supply nutrients necessary for its continued development.

Unraveling the phenomenon of angiogenesis has potential application in controlling the unrestrained growth of tumors and providing treatment for vascular disorders. Further, the growth factors have potential use in the vascularization of damaged or implanted tissue.

To understand angiogenesis, endothelial cell growth factors from normal tissue have been isolated. One isolated endothelial cell growth factor is fibroblast growth factor (FGF), a normal endothelial cell mitogen found both in pituitary and brain cells. The FGF has recently been purified to homogeneity. The amino terminal sequence of bovine brain-derived FGF is reported in D. Gospodarowicz et al., Proc. Natl. Acad. Sci. USA 81:6963 (1984). The complete amino acid sequence of human brain-derived FGF is described in Gimenez-Gallego et al., Science 230:1385-1389 (1985). The pituitary bovine FGF amino terminal sequence is described in Bohlen et al., Proc. Natl. Acad. Sci. USA 81:5364 (1984). The complete amino acid sequence of FGF from bovine pituitary cells is reported in Esch et al., Proc. Nat'l Acad. Sci. USA 82:6507 (1985). The amino terminal sequence of human brain FGF is described in Bohlen et al., J. Cell. Biochem. Supplement 9a:133 (1985). The amino-terminal sequences of two forms of human brain FGF is reported in Bohlen et al., FEBS 185:177 (1985).

Shing et al., Science 223:1296-1299 (1984), reports the isolation and apparent purification of a chondrosarcoma-derived growth factor from a malignant cartilage tumor. In the Shing study, after isolating this factor, it was applied to a BioRex 70 cation-exchange column. The growth factor activity peak obtained by BioRex 70 chromatography was applied to a Heparin-Sepharose column.

## Description of the Invention

Hepatoma-derived growth factor (HDGF) from hepatoma cells was isolated and applied to the two-step BioRex 70 affinity chromatography and Heparin-Sepharose column as described in Shing et al., supra. The inventor herein discovered that the HDGF material obtained by this two-step

process was, however, only incompletely pure. Upon having discovered this problem of this two-step process, the inventor then solved the additional problem of providing a material having HDGF activity and being substantially free of proteinaceous impurities.

The invention therefore relates to the purification to homogeneity of a tumor-derived endothelial cell growth factor from hepatoma cells, a hepatoma-derived growth factor substantially free of any proteinaceous impurities having molecular weight less than about 18,000 daltons. The invention also relates to the identification and characterization of the hepatoma-derived growth factor. The invention further relates to uses of the hepatoma-derived growth factor.

The inventor has purified to homogeneity a tumor-derived endothelial cell growth factor from hepatoma cells. The homogeneous hepatoma-derived growth factor (HDGF) is characterized by a peak of biological activity, derived from reverse-phase HPLC chromatography. The inventor has discovered that the hepatoma-derived growth factor (HDGF) is structurally related to fibroblast growth factor (FGF). The homology between HDGF and FGF has implications for understanding and controlling angiogenesis and certain vascular disorders.

## Brief Description of the Drawings

Figure 1 is a graphic representation of the results of an evaluation of various elution fractions of HDFG from Heparin-Sepharose purification with regard to their ability to stimulate DNA synthesis in BALB c/3T3 cells (open circles) and proliferation of capillary endothelial cells (closed circles). The Inset depicts the results of an SDS-PAGE and silver stain analysis of the biologically active fractions,

showing the molecular weight of the active fraction to be approximately 18.5 kilodaltons.

Figure 2 is a graphic representation of an evaluation of 1 ml elution fractions (abscissa) of HDGF, from the Heparin-Sepharose column on a reverse-phase HPLC with a 0-60% linear gradient of acetonitrile/isopropanol (50/50 v/v) in 0.1% trifluoroacetic acid (right ordinate) with regard to their ability to stimulate DNA synthesis in BALB c/3T3 cells (open circles, left ordinate) and proliferation of capillary endothelial cells (closed circles, left ordinate) and proliferation of capillary endothelial cells (closed circles, left ordinant). The inset depicts the results of an SDS-PAGE and silver stain analysis of the biologically active fractions, showing the molecular weight of the active fractions to be approximately 18,500 daltons. Figure 2 also shows the UV absorbance at 214 nm of the elution fractions.

Figure 3A is a graphic representation of the results of an evaluation of 1 ml elution fractions (abscissa) of HDGF from the Heparin-Sepharose column on a reverse-phase HPLC with a 0-60% linear gradient of acetonitrile/isopropanol (50/50 v/v) in 0.1% trifluoroacetic acid (right ordinate) with regard to their ability to stimulate DNA synthesis in BALB c/3T3 cells (closed circles, left ordinate) and their immunoreactivity in an immuno-dot assay (open circles, left ordinate). In the immuno-dot assay, the intensity of the "dots" was quantitated by reflectance densimetry and plotted on an arbitrary scale of from 0 (no immunoreaction, 100% reflectance) to 1 (maximal immunoreaction, 0% reflectance).

Figure 3B is a Western blot analysis of HDGF purified by BioRex and Heparin-Sepharose chromatography (Lanes 1 and 3) and purified by BioRex 70 chromatography alone (Lanes 2 and 4). Lanes 1 and 2 were stained for protein; Lanes 3 and 4

were incubated with anti-FGF antiserum and visualized by reflectance densimetry as described in Figure 3A.

Figure 4 shows a comparison of the sequence of a hepatoma-derived growth factor peptide fragment with the amino-terminal sequences of bovine fibroblast growth factor and human fibroblast growth factor.

## Best Mode of Carrying out the Invention

### I. Isolation, Purification, and Sequencing of the Hepatoma-Derived Growth Factor

In accordance with this invention, an endothelial cell growth factor from hepatoma cells was purified to homogeneity. Any sample that contains the factor may be used as a starting material according to the method described by this invention. Typically, the hepatoma-derived growth factor may be isolated from a human or animal hepatoma or from hepatoma cell lines.

In one embodiment, a human hepatoma cell line SK-HEP 1, described by J. Fogh and G. Trempe in Human Tumor Cells in Vitro, J. Fogh, Editor (New York, 1975), p. 115, was used as the starting material to isolate the factor. It will be understood by one of skill in the art that other sources of starting material may be used to obtain the hepatoma-derived growth factor. This human hepatoma cell line, SK-HEP 1, was grown in suspension culture. Most of the growth factor activity was found to be associated with the cells rather than with the conditioned medium. Accordingly, the hepatoma cells were lysed by a succession of freezing, thawing, and homogenization to recover the HDGF.

The isolated crude HDGF was first applied to a combination of chromatography steps using BioRex 70 cation exchange

and Heparin-Sepharose affinity chromatography. These purification steps followed the procedures described in Shing et al., Science 223:1296-1299 (1984). Crude hepatoma lysate was applied to BioRex 70 and HDGF was eluted with 0.6M NaCl. The active fractions were pooled and applied directly to a column of Heparin-Sepharose (Figure 1). One peak of growth factor activity, as measured by the ability of fractions to stimulate 3T3 cell DNA synthesis (Thomas, K.A., et al., J. Biol. Chem. 255:5517 (1980)) and capillary endothelial cell proliferation (Klagsbrun, M., et al., Proc. Natl. Acad. Sci. USA 82:805 (1985)) eluted at about 1.8M NaCl. Analysis of the active fractions by SDS-PAGE and silver stain revealed the presence of a band with a molecular weight of about 18,500 daltons (Figure 1, inset). The HDGF had a specific activity of about 5 units/ng and stimulated cell proliferation at about 1 ng/ml. A unit of growth factor activity was defined as the concentration of growth factor needed to stimulate half maximal stimulation of DNA synthesis in 3T3 cells. About 2-4 x $10^4$ hepatoma cells were found to contain 1 unit of HDGF. The recovery of HDGF was about 30%.

It had been previously reported that the noted two-step chromatography application resulted in purification of a tumor-growth factor, Shing et al., supra. However, the applicant found that HDGF material obtained by this combination was not homogeneous, but a mixture of the HDGF and proteinaceous impurities. When the HDGF SDS-PAGE gels were overloaded with material from the two-step chromatography, the gel profile showed quite a number of protein impurities between molecular weight 3,000 and 10,000 daltons. Upon having discovered that the HDGF material was not purified to homogeneity by the two-step chromatography process, the applicant then addressed the additional problem of providing

a material having HDGF activity and being truly substantially free of proteinaceous impurities.

The Heparin-Sepharose purified material was injected into a reverse-phase high pressure liquid chromatography (HPLC) column with a C3 packing, and eluted with 0.1% trifluoroacetic acid (TFA) in an acetonitrile gradient. Reverse-phase chromatography resulted in the partial resolution of two peaks of biologically active HDGF eluting between 32 and 37% organics (acetonitrile and isopropanol: 50:50 (volume/volume)). The right-hand peak of biological activity as shown in Figure 2, from fractions 34 to 37, represents the homogeneous HDGF. Thus, after Heparin-Sepharose purification, the HDGF is purified to homogeneity by applying the HDGF to a reverse-phase HPLC column.

As is known in the art, reverse-phase HPLC under the above conditions results in at least partial denaturing of the protein. In order to regain biological activity, the denatured HDGF may be renatured according to means known in the art, such as neutralizing the elution solution containing the HDGF by adding a neutralizing buffer. Alternatively, to preserve the biological activity of the HDGF during the reverse-phase chromatography step, a two-component buffer may be used, such as $Na_2HPO_4/NaH_2PO_4$ or sodium acetate/acetic acid. Other suitable buffer solutions are known in the art and may be used in this invention. For industrial or large-scale purification or separation techniques of HDGF, isocratic conditions can be used with step-wise increase in organic solvent concentration. Under isocratic conditions, the organic and aqueous compositions are kept constant, as opposed to a gradient of changing compositions.

The HDGF represented by the right-hand peak of biological activity in Figure 2, between fractions 34 to 37, was

then concentrated and used to obtain amino acid sequencing information.

As used herein, the term "substantially pure" or "substantially purified" is meant to describe the HDGF which is substantially free of any compound normally associated with the factor in its natural state: free of other proteins and of carbohydrate or lipid components. The term is further meant to describe the factor which is homogeneous by one or more purity or homogeneity characteristics used by those of skill in the art. For example, a substantially pure HDGF will show constant and reproducible characteristics within standard experimental deviations for parameters such as the following: molecular weight, chromatographic techniques, amino acid composition, amino acid sequence, blocked or unblocked N-terminus, HPLC elution profile, biological activity, and such other parameters. The term, however, is not meant to exclude artificial or synthetic mixtures of the factor with other compounds. In particular, as used herein, the term "substantially pure" or "substantially purified" or "substantially free of" is meant to describe HDGF purified to homogeneity such that there is substantially no proteinaceous impurity associated with the HDGF, and the HDGF is substantially free of proteinaceous material having molecular weights of less than about 18,000 daltons.

## II. Structural Homology Between the Hepatoma-Derived Growth Factor and Fibroblast Growth Factor

The structural relationship was compared between the hepatoma-derived growth factor of the invention, which is a tumor-derived endothelial cell growth factor, and fibroblast growth factor (FGF), a normal tissue endothelial cell growth factor described in Gimenez-Gallego et al., Science,

230:1385-1389 (1985); P. Bohlen et al., J. Cell. Biochem. Supplement, 9a:133 (1985); D. Gospodarowicz et al., Proc. Nat'l Acad. Sci. USA, 81:6963 (1984); P. Bohlen et al., Proc. Nat'l Acad. Sci. USA, 81:5364 (1984); and Esch et al., Proc Nat'l Acad. Sci. USA, 82:6507 (1985).

A synthetic peptide corresponding to the 15 amino acid amino terminal sequence of bovine FGF, shown in Figure 4, was conjugated to the immunogenic carrier keyhole limpet hemocyanin (KLH) and injected into rabbits. The polyclonal antibodies that were produced were tested for their ability to cross-react with HDGF (Figures 3A and 3B) after purification by HPLC on a reverse-phase C3 column. The fractions that were eluted from the column were tested for growth factor activity and for ability to cross-react with anti-FGF antisera in an immuno-dot-blot test (Figure 3A). Only those fractions containing biologically active HDGF were found to cross-react with anti-FGF antisera.

The cross-reactivity of anti-FGF antisera for HDGF was further analyzed by use of a "Western Blot" assay (Figure 3B). Both crude and homogeneous preparations of HDGF were electrophoresed by SDS-PAGE and the proteins were blotted onto nitrocellulose by electrophoretic transfer. The transferred proteins were stained for protein (lanes 1 and 2) and were incubated with anti-FGF antiserum (lanes 3 and 4). Purified 18,500 molecular weight HDGF (lane 1) was clearly immunoreactive with the antisera prepared against synthetic FGF (lane 3). The antisera could be shown to be highly specific in its ability to detect HDGF in a crude mixture of protein. Of the many proteins found in the crude preparation of HDGF (lane 2), only a polypeptide doublet with molecular weights of about 18,000-19,000 was clearly immunoreactive with anti-FGF antiserum (lane 4). The nature of the doublet

is unclear but still suggests the presence of different forms of HDGF in the crude preparation.

It could be concluded from the antibody experiments that HDGF contains a sequence homologous to the amino terminal sequence of FGF. However, whether the amino terminal sequence of FGF is also the amino terminal of HDGF could not be ascertained from these experiments. Accordingly, HDGF was purified on a large scale and an attempt was made to determine its amino acid sequence using an Applied Biosystems 470A Sequenator. When 5 nmoles (about 100 ug) of HDGF were submitted for sequence analysis, no sequence data was obtained, suggesting that HDGF was blocked at the N-terminal amino acid. The result obtained in the present case suggested that the N-terminal regions of HDGF and FGF were different.

Consequently, HDGF was cleaved with trypsin and the fragments were separated on an HPLC phenyl reverse phase column (The Separations Group, Hesperia, California). A number of the fragments were sequenced. One of the sequences was found to be leu-pro-ala-leu-pro-glu-asp-gly-gly-X-gly-ala-phe-pro-pro-gly (Figure 4), wherein X is an unidentified amino acid moiety. Comparison of this human HDGF sequence with that of the amino terminal sequences of bovine and human FGF is shown in Figure 4. Starting with its third amino acid residue (pro) the sequence of the HDGF fragment was found to be homologous with the 15 amino acid amino terminal sequence of bovine FGF. The HDGF sequence shows the same homology with human FGF except that human FGF has a glu residue at its position 6. It could be concluded from the sequence data that HDGF contains within it the 15 amino acid terminal sequence of FGF. However in HDGF, this sequence is internal and contains at least two additional amino acids on the amino terminal side. Furthermore, the blocked nature of the HDGF

N-terminus suggests that HDGF contains more than two additional amino acids that are N-terminal to the amino terminal FGF sequence. The N-terminal extension sequence for HDGF comprises the following amino acid sequence: (ala/ser)-(leu/arg)-pro-(ala/gly)-(leu/pro)-ala-gly-thr-met-ala-(ala)-gly-ser-(isoleu)-thr-thr-leu.

The antibody cross-reactivity and the protein sequence data make it clear that there is some degree of structural homology between HDGF and FGF. Strong similarities in other properties such as chromatographic behavior on BioRex 70 and on Heparin-Sepharose also suggest structural homology.

The homology between HDGF and FGF has implications for the mechanism of angiogenesis and its control. Both tumor-derived endothelial cell growth factors and FGF induce angiogenesis. The structural homology between HDGF and FGF suggests that structurally similar proteins may be involved in both tumor-induced angiogenesis and in the angiogenesis that occurs normally during development. Thus, vascularization of a tumor may result from the abnormal production of a normally occurring protein.

III. Uses of the Hepatoma-Derived Growth Factor

The substantially purified hepatoma-derived growth factor has clinical utility in several areas. The HDGF can be used to stimulate the production of antibodies. In addition, peptide fragments of the substantially purified HDGF which are both immunogenic and immunospecific can also be used to raise antibodies.

Of particular interest are HDGF peptides of the following formula:

1) $H_2N$--X--CO--$R^1$

wherein $R^1$ is Cys-CO-$R^2$, OH, OM or -$NR^3R^4$;

M is a pharmaceutically acceptable cation or a lower ($C_1$-$C_6$) branched or unbranched alkyl group;

$R^2$, $R^3$ and $R^4$ are the same or different and selected from the group consisting of hydrogen and a lower ($C_1$-$C_6$) branched or unbranched alkyl group; and

X is the immunogenic and immunospecific HDGF peptide fragment as described in Figure 4 or another immunogenic and immunospecific peptide fragment of HDGF;

2) the acid addition salts thereof; and

3) the protected or partially protected derivatives thereof.

As is known in the art, the amino acid residues may be in their protected or unprotected form, using appropriate amino or carboxyl protecting groups.

Useful cations M are alkali or alkaline earth metallic cations (i.e., Na, K, Li, 1/2 Ca, 1/2 Ba, etc.) or amine cations (i.e., tetraalkylammonium, trialkylammonium, where alkyl can be $C_1$-$C_{12}$).

The variable length peptides may be in the form of the free amines (on the N-terminus), or acid-addition salts thereof. Common acid addition salts are hydrohalic acid salts, i.e., HBr, HI, or more preferably, HCl.

The immunogenic acid immunospecific peptide fragments of HDGF may be determined according to means known in the art. For example, the HDGF may be cleaved into peptide fragments and the amino acid sequence of the peptide fragments determined. In the preferred embodiment, peptide fragments may be synthesized by the well known solid phase synthesis described by Merrifield, J. Am. Chem. Soc., 85:2149 (1962) and Stewart and Young, in Solid Phase Peptide Synthesis (Freeman, San Francisco, 1969) pp. 27-62, incorporated by reference herein. Various peptide fragments can then be evaluated to determine immunogenicity (the property that endows a substance with the

capacity to provoke a humoral immune response and the degree to which the substance possesses this property) and immunospecificity (the ability of the antibodies provoked by the immune response to bind to the HDGF or HDGF peptide fragment). These peptide fragments are then utilized in procedures known to the art to determine the immunogenicity and immunospecificity, by evaluating the peptide fragments ability to stimulate antibody production.

To produce antibodies, the HDGF or peptide fragment may be coupled to a carrier protein such as albumin or keyhole limpet hemocyanin (KLH), utilizing techniques well known and commonly used in the art. Additionally, the HDGF or peptide fragment can be mixed with an immunologically inert or active carrier. Carriers which promote or induce immune responses, such as Freund's complete adjuvant, can be utilized. The antigenic material (HDGF or peptide fragment-carrier protein conjugate) is introduced into the immune system of the animal in which antibodies are to be raised. Both polyclonal antibodies and monoclonal antibodies, produced by well-known techniques, raised in response to the HDGF or immunogenic peptide sequences of the HDGF can be utilized in various ways.

These antibodies may be utilized in immunoassays to identify and quantitate the hepatoma-derived growth factor. The immunoassays within the scope of the present invention include both the sandwich technique and the competitive assay of enzyme-linked immunosorbent assay (ELISA). These assays are well known in that art and are described, for example, in Voller, A. et al., The Enzyme Linked Immunosorbent Assay (ELISA), (Dynatech Europe 1979). As will also be understood by those of skill in the art, use of these immunodiagnostic assays involves antibodies raised in response to the HDGF or an immunogenic peptide fragment of HDGF. The assays also

involve detectably labeling the peptide fragments and antibodies. Techniques for producing antibodies and detectably labeling peptide fragments and antibodies are well known in the art and will be known by one of skill in the art.

In addition, the materials for use in the assays of the invention are ideally suited for preparation of a kit. Such a kit may comprise a carrier means being compartmentalized to receive in close confinement one or more container means such as vials, test tubes, and the like. Each of said container means comprises one of the separate elements to be used in the method.

For example, one of said container means may comprise an antibody against HDGF as described above. Such fragment may be bound to a separate solid phase immunoabsorbent or directly to the inner walls of a container. A second container may comprise detectably labeled anti-antibody in lyophilized form or in solution.

The carrier may also contain, in addition, a plurality of containers each of which comprises different, predetermined known amounts of antibody. These latter containers can then be used to prepare a standard curve from which can be interpolated the results obtained from the sample containing the unknown amount of HDGF.

These antibodies may also be utilized to treat a patient suffering from angiogenesis of liver tumor cancer cells (hepatoma), by administering a therapeutically effective amount of the polyclonal antibodies, or preferably the monoclonal antibodies, to the patient.

The HDGF or therapeutic fragments thereof can be utilized to treat patients with damaged tissues or implanted tissue. These types of tissue will typically be in need of vascularization to aid in healing. Thus, the HDGF or

therapeutic fragments thereof can be administered to aid in the growth of new blood supply. Typically, the HDGF or therapeutic fragments thereof will be administered in a pharmaceutical composition comprising a neutral or inert carrier and the HDGF or therapeutic fragments of HDGF. The therapeutic fragments of HDGF will comprise any fragment which can promote the growth of new blood vessels. Preferably, these fragments will be the HDGF peptide fragments as shown in Figure 4.

The following examples describe the materials and methods used in carrying out the invention. The examples are not intended to limit the invention in any manner.

## EXAMPLE 1

Figure 1 shows the isolation and partial purification of hepatoma-derived growth factor (HDGF). Human hepatomas SK-HEP-1 cells were grown in suspension as described in W.R. Tolbert et al., Biotech. and Bioeng., 24:1671 (1982). About $5 \times 11^{10}$ cells were collected by centrifugation. The pellet, about 150 ml, was frozen, thawed and resuspended in 1500 ml of 1 M NaCl, 0.01 M Tris-HCl, pH 7.5. The hepatoma cells were disrupted by homogenization in a Waring Blender for 1 minute at room temperature. The homogenate was stirred overnight at 4 degrees C and subsequently clarified by centrifugation at 25,000g for 30 minutes. The clarified supernatant was dialyzed against 0.015 M NaCl, 0.01 Tris-HCl and mixed with 1750 ml of BioRex 70 (BioRad, 200-400 mesh), equilibrated with the same buffer, by stirring overnight at 4 degrees C. The BioRex 70 was collected, poured into a column (5 x 100 cm), washed with 2000 ml of equilibration buffer and then with 2000 ml of 0.6 M NaCl, 0.01 M Tris-HCl, pH 7.5 at a flow rate of 60 ml/hour at 4 degrees C. Fractions were collected

and monitored for the ability to stimulate DNA synthesis in BALB/c 3T3 cells and proliferation of capillary endothelial cells as previously described in K.A. Thomas et al., J. Biol. Chem., 255:5517 (1980) and M. Klagsbrun et al., Proc. Natl. Acad. Sci. U.S.A., 82:805 (1985). Fractions containing HDGF were pooled (about 300 ml, 1.5 x $10^6$ growth factor activity units as defined in M. Klagsbrun et al., Proc. Natl. Acad. Sci. U.S.A., 82:805 (1985)) and applied directly to a column of Heparin-Sepharose (Pharmacia, 2 x 13 cm, 40 ml) equilibrated with 0.6 M NaCl, 0.01 M Tris-HCl, pH 7.5. After a wash of 200 ml with equilibration buffer, a 400 ml gradient of 0.6 M NaCl, 0.01 M Tris-HCl, pH 7.5 was applied to the column at a flow rate of 40 ml/hour at 4 degrees C. Fractions (7 ml) were collected and tested for conductivity and for the ability to stimulate DNA synthesis in 3T3 cells and proliferation of capillary endothelial cells. The results are reported at Figure 1, with the closed circles representing DNA synthesis, measured in counts per minute times $10^{-4}$, and the open circles representing capillary endothelial cell proliferation, measured as endothelial cell number times $10^{-4}$. Referring to Figure 1 it may be seen that fractions 28-32 demonstrate the peak of HDGF biological activity.

The Insert of Figure 1 shows the molecular weight of the fractions. About 10% of each of the active fractions (28-31) were dialyzed against distilled $H_2O$, lyophilized and analyzed by SDS-polyacrylamide gel electrophoresis (Laemmli, Nature, 277:680 (1970)) and silver stain (Oakley et al., Anal. Biochem., 105:362 (1975)). The biologically active protein has a molecular weight of about 18,500.

## Example 2

Figure 2 shows the reverse-phase chromatography of HDGF. HDGF isolated and partially purified by a combination of BioRex 70 and Heparin-Sepharose chromatography (Example 1) was desalted by reverse-phase high performance liquid chromatography (HPLC) using a Beckman model 334 HPLC gradient system. HDGF (about 400,000 units, 80 ug, 60 ml) was applied to an RPSC C3 column (0.46 x 7.5 cm, Beckman Instruments) via a separate minipump (LDC/Miniroy pump) at a flow rate of 1.0 ml/min at room temperature. After a wash with 0.1% trifluoroacetic acid (TFA), a 60 ml 0-60% linear gradient of acetonitrile/2-propanol (50/50 v/v) in 0.1% TFA was applied to the column at a flow rate of 0.5 ml/minute and fractions (1 ml) were collected. For measurements of biological activity, aliquots from each fractions were quickly diluted about 20-fold into phosphate buffered saline supplemented with 1 mg/ml serum albumin in order to neutralize the pH. The results are reported in Figure 2. The closed circles indicate the stimulation of DNA synthesis in 3T3 cells. The open circles indicate the stimulation of proliferation of capillary endothelial cells. Figure 2, Insert, shows the molecular weight of the biologically active fractions to be approximately 18,500 daltons. In making this molecular weight determination, fractions 32-37 were pooled and dried in a speevac concentrator (Savant Instruments). About 800 ng of HDGF were analyzed by SDS-PAGE and silver staining.

## Example 3

Figure 3 shows the immunodetection of HDGF with antibodies prepared against the amino terminal sequence of bovine FGF.

A peptide corresponding to the 15 amino acids on the amino terminal side of bovine FGF (D. Gospodarowicz et al., Proc. Natl. Acad. Sci. U.S.A., 81:6963 (1984)) was synthesized by the solid-phase method of Merrifield (R.B. Merrifield, J. Am. Chem. Soc., 85:2149 (1963)) using an automated Applied Biosystems 430. A peptide synthesized, t-butoxycarbonyl (Boc)-gly, was coupled to hydroxymethyl-phenylacetaminodo-methyl(Pam)-polystyrene resin and peptide assembly was carried out using Boc-amino acids (Peninsula Laboratories) to produce 1 gram of Boc-pro-ala-leu-pro-glu-asp(OBzl)-gly-gly-ser-(OBzl)-gly-ala-phe-pro-pro-gly-OCH2-Pam-resin. The resin (0.97 g) was treated with 10 ml anhydrous hydrogen fluoride in the presence of 1.0 ml p-cresol and 1.0 g p-thiocresol for 1 hour at 0 degree C and extracted with diethyl ether and 30% glacial acetic acid. The peptide was desalted on a Sephadex G-25 column (2.5 x 90 cm) equilibrated with 1 M glacial acetic acid and fractionated on an HPLC Vydac C4 reverse phase column (0.46 x 25 cm) using a linear gradient of 0-60% acetonitrile in 0.1% TFA. The amino acid composition of the synthetic peptide corresponded to that of the amino terminal sequence of bovine FGF. The synthetic FGF peptide was conjugated to keyhole limpet hemocyanin (KLH) using 1-ethyl-3-(3-dimethylaminopropyl) carbodiimide. Rabbits were immunized by multisite intradermal injections of KLH-peptide conjugate (500 ug) emulsified with complete Freund's adjuvant followed 3 and 6 weeks later with subcutaneous injections of 200 ug of KLH-peptide conjugate emulsified in incomplete Freund's adjuvant. The titer of the antisera after the second booster injection was about 1:4000 - 1:9000 as determined in an ELISA-assay using unconjugated FGF peptide as the antigen.

A. Immuno-dot assay. HDGF was purified by a combination of BioRex 70 and Heparin-Sepharose chromatography as

described in Example 1 and C3 reverse phase chromatography as described in Example 2. Each fraction collected after reverse phase chromatography was tested for growth factor activity on 3T3 and for immunoreactivity in an immuno-dot assay. The results are reported at Figure 3A, with the closed circles representing DNA synthesis in BALB c/ 3T3 cells, and the open circles representing immunoreactivity. In the immuno-dot assay, 2 ul of each column fraction was diluted with 100 ul of distilled $H_2O$ and filtered through 96 well microfiltration manifolds (Bio-dot apparatus, Bio-Rad laboratories) onto BA-83 0.2 um nitrocellulose sheets (Schleicher and Schuell). The protein "dots" were incubated with affinity purified anti-FGF antiserum (1:1500) and bound immunoglobulin was visualized by successive incubations with biotinylated goat anti-rabbit antibodies, peroxidase-conjugated streptavidin and 4-chloro-1-napthol substrate. The intensity of the "dots" was quantitated by reflectance densitometry and plotted on an arbitrary scale from 0 (no immunoreaction, 100% reflectance) to 1 (maximal immunoreaction, 0% reflectance).

B. Western blot analysis. Proteins were electrophoresed on an 18% SDS-polyacrylamide gel and transferred electrophoretically to BA-83 nitrocellulose paper (Trans-Blot cell, Bio-Rad). Portions of nitrocellulose paper were either stained for protein with "Aurodye colloidal gold reagent" (Janssen Life Sciences Products) or incubated with anti-FGF antiserum and visualized as described in the immuno-dot assay of Example 3A. The results are reported in Figure 3B. Replicate samples of HDGF purified by BioRex and Heparin-Sepharose chromatography were stained for protein (lane 1) and stained with anti-FGF antiserum (lane 3). Replicate samples of crude HDGF partially purified by BioRex 70

chromatography alone were stained for protein (lane 2) and stained for antibody (lane 4).

Example 4

Figure 4 compares the sequence of an HDGF peptide fragment with that of the amino terminal sequences of bovine and human FGF. HDGF was purified by a combination of BioRex 70, Heparin Sepharose and C3 reverse phase chromatography as described in Examples 1 and 2. After reverse phase chromatography, HDGF (about 27 ug) was dried in a Speedvac concentrator, resuspended in 0.5 ml of 0.2 M ammonium bicarbonate buffer and digested with trypsin (Cooper Biomedical), TPCK treated, (enzyme to substrate ratio of 1/40, wt/wt) at 37°C for 8 hours. The digest was dried in a Speedvac concentrator, dissolved in 0.2 ml 6M guanidine-HCl, 0.1% TFA and applied to a Vydac HPLC reverse phase phenyl column (The Separations Group, 0.46 x 25 cm). The tryptic fragments were separated with a 120 ml linear gradient of 0-60% acetonitrile in 0.1% TFA at a flow rate of 1 ml/minute. The peptide peak was collected, dried in a Speedvac concentrator, dissolved in 0.1 ml of 0.5 TFA/0.1% SDS, applied to a cartridge filter treated with polybrene and submitted to an Applied Biosystems 470A sequenator for sequence analysis as described in M.W. Hunkapillar et al., Methods in Enzymology, 91:486 (1968).

The sequence of a 17 amino acid fragment of HDGF is shown in the first row. Parentheses surrounding an amino acid residue (abbreviated in three letters) indicate that the residue was the predominant one in the Edman degradation cycle. Parentheses without an amino acid residue included indicates that no amino acid residue could be definitely identified. Row 2 contains the published amino terminal sequence of bovine FGF (D. Gospodarowicz et al., Proc. Natl.

Acad. Sci. U.S.A., 81:6963 (1984) and Esch et al., Proc. Natl. Acad. Sci. U.S.A., 82:6507 (1985)). Row 3 contains the published amino terminal sequence of human FGF (P. Bohlen et al., J. Cell. Biochem. (abstract) Supplement 9a:133 (1985)).

Having now fully described this invention, it will become readily apparent to one with ordinary skill in the art that many changes and modifications may be made thereto without affecting the spirit or scope thereof.

CLAIMS:

1. Hepatoma-derived growth factor (HDGF) substantially free of any proteinaceous impurities with molecular weights of less than about 18,000 daltons, said HDGF having a molecular weight of about 18,500 daltons.

2. A hepatoma-derived growth factor peptide fragment having an N-terminal amino acid sequence wherein the first sixteen amino acids are:

leu-pro-ala-leu-pro-glu-asp-gly
gly-X-gly-ala-phe-pro-pro-gly

wherein X is an unidentified amino acid moiety.

3. Hepatoma-derived growth factor (HDGF) substantially free of any proteinaceous impurities with molecular weights of less than about 18,000 daltons, said HDGF obtainable by a process comprising:

i) recovering crude hepatoma-derived growth factor (HDGF) from a hepatoma;
ii) subjecting said crude HDGF from step (i) to cation exchange to obtain partially purified active fractions of HDGF defined as the concentration of growth factor needed to stimulate half-maximal stimulation of DNA synthesis in 3T3 cells;
iii) applying said partially purified active fractions of HDGF from step (ii) to Heparin-Sepharose affinity chromatography to obtain HDGF; and
iv) purifying HDGF to homogeneity by applying said HDGF from step (iii) to reverse-phase high pressure

liquid chromatography to obtain HDGF substantially free of any proteinaceous impurities with molecular weights of less than about 18,000 daltons.

4. An antibody derived by using an immunogen comprising the hepatoma-derived growth factor of claim 1.

5. An antibody derived by using an immunogenic and immunospecific peptide fragment of the hepatoma-derived growth factor of claim 1.

6. An antibody derived by using an immunogen comprising the peptide fragment of claim 2.

7. An antibody according to claim 4, 5, and 6 in labeled from.

8. A pharmaceutical composition comprising a neutral carrier and the hepatoma-derived growth factor of claim 1.

9. A pharmaceutical composition comprising a neutral carrier and the hepatoma-derived growth factor peptide fragment of claim 2.

10. A peptide having the formula:

1) $H_2N--X--CO--R^1$

wherein $R^1$ is Cys-CO-$R^2$, OH, OM or -$NR^3R^4$;

M is a pharmaceutically acceptable cation or a lower ($C_1$-$C_6$) branched or unbranched alkyl group;

$R^2$, $R^3$ and $R^4$ are the same or different and selected from the group consisting of hydrogen and a lower ($C_1$-$C_6$) branched or unbranched alkyl group; and

X is an immunogenic and immunospecific peptide fragment of the hepatoma-derived growth factor of claim 1 or the peptide fragment of claim 2;

2) the acid addition salts thereof; and

3) the protected or partially protected derivatives thereof.

11. A method for detecting hepatoma-derived growth factor in a sample comprising contacting said sample suspected of containing hepatoma-derived growth factor (HDGF) with antibodies to said HDGF, and detecting the HDGF by measuring the formation of antigen-antibody complexes using an immunodiagnostic assay.

12. A hepatoma-derived growth factor peptide fragment having an N-terminal amino acid extension sequence comprising:

(ala/ser)-(leu/arg)-pro-(ala/gly)-(leu/pro)-ala-gly-thr-met-ala-(ala)-gly-ser-(isoleu)-thr-thr-leu.

13. A process for preparing a hepatoma-derived growth factor (HDGF) substantially free of any proteinaceous impurities with molecular weights of less than 18,000 daltons, comprising:

i) recovering crude hepatoma-derived growth factor (HDGF) from a hepatoma;

ii) subjecting said crude HDGF from step (i) to cation exchange to obtain partially purified active fractions of HDGF defined as the concentration of growth factor needed to stimulate half-maximal stimulation of DNA synthesis in 3T3 cells;

iii) applying said partially purified active fractions of HDGF from step (ii) to Heparin-Sepharose affinity chromatography to obtain HDGF; and

iv) purifying HDGF to homogeneity by applying said HDGF from step (iii) to reverse-phase high pressure liquid chromatography to obtain HDGF substantially free of any proteinaceous impurities with molecular weights of less than about 18,000 daltons.

14. The process of claim 13, wherein said HDGF has a molecular weight of about 18,500 daltons.

15. A process for preparing a HDGF peptide fragment having an N-terminal amino acid sequence in which the first 16 amino acids are:

leu-pro-ala-leu-pro-glu-asp-gly
gly-X-gly-ala-phe-pro-pro-gly

with X being an unidentified amino acid moiety, which comprises cleaving HDGF with trypsin and separating the fragments on an HPLC phenyl reverse phase column.

16. The process of claim 15, wherein said peptide fragment has an N-terminal amino acid extension sequence comprising:

(ala/ser)-(leu/arg)-pro-(ala/gly)-(leu/pro)-ala-gly-
thr-met-ala-(ala)-gly-ser-(isoleu)-thr-thr-leu.

17. A process for preparing an antibody which comprises using the HDGF obtained according to claim 13 or 14 or a

fragment obtained according to claim 15 or 16 as an immunogen.

18. The process of claim 17 wherein an immunogenic and immunospecific peptide fragment of the HDGF obtained according to claim 15 or 16 is used.

19. A method of detecting HDGF in a sample comprising contacting said sample suspected of containing HDGF with antibodies to said HDGF, and detecting the HDGF by measuring the formation of antigen-antibody complexes using an immunodiagnostic assay.

Patent Claims for Austria, Greece and Spain:

1. A process for preparing a hepatoma-derived growth factor (HDGF) substantially free of any proteinaceous impurities with molecular weights of less than 18,000 daltons, comprising:

i) recovering crude hepatoma-derived growth factor (HDGF) from a hepatoma;

ii) subjecting said crude HDGF from step (i) to cation exchange to obtain partially purified active fractions of HDGF defined as the concentration of growth factor needed to stimulate half-maximal stimulation of DNA synthesis in 3T3 cells;

iii) applying said partially purified active fractions of HDGF from step (ii) to Heparin-Sepharose affinity chromatography to obtain HDGF; and

iv) purifying HDGF to homogeneity by applying said HDGF from step (iii) to reverse-phase high pressure liquid chromatography to obtain HDGF substantially free of any proteinaceous impurities with molecular weights of less than about 18,000 daltons.

2. The process of claim 1, wherein said HDGF has a molecular weight of about 18,500 daltons.

3. A process for preparing a HDGF peptide fragment having an N-terminal amino acid sequence in which the first 16 amino acids are:

leu-pro-ala-leu-pro-glu-asp-gly
gly-X-gly-ala-phe-pro-pro-gly

with X being an unidentified amino acid moiety, which comprises cleaving HDGF with trypsin and separating the fragments on an HPLC phenyl reverse phase column.

4. The process of claim 3, wherein said peptide fragment has an N-terminal amino acid extension sequence comprising:

(ala/ser)-(leu/arg)-pro-(ala/gly)-(leu/pro)-ala-gly-thr-met-ala-(ala)-gly-ser-(isoleu)-thr-thr-leu.

5. A process for preparing an antibody which comprises using the HDGF obtained according to claim 1 or 2 or a fragment obtained according to claim 3 or 4 as an immunogen.

6. The process of claim 5 wherein an immunogenic and immunospecific peptide fragment of the HDGF obtained according to claim 3 or 4 is used.

7. A method of detecting HDGF in a sample comprising contacting said sample suspected of containing HDGF with antibodies to said HDGF, and detecting the HDGF by measuring the formation of antigen-antibody complexes using an immunodiagnostic assay.

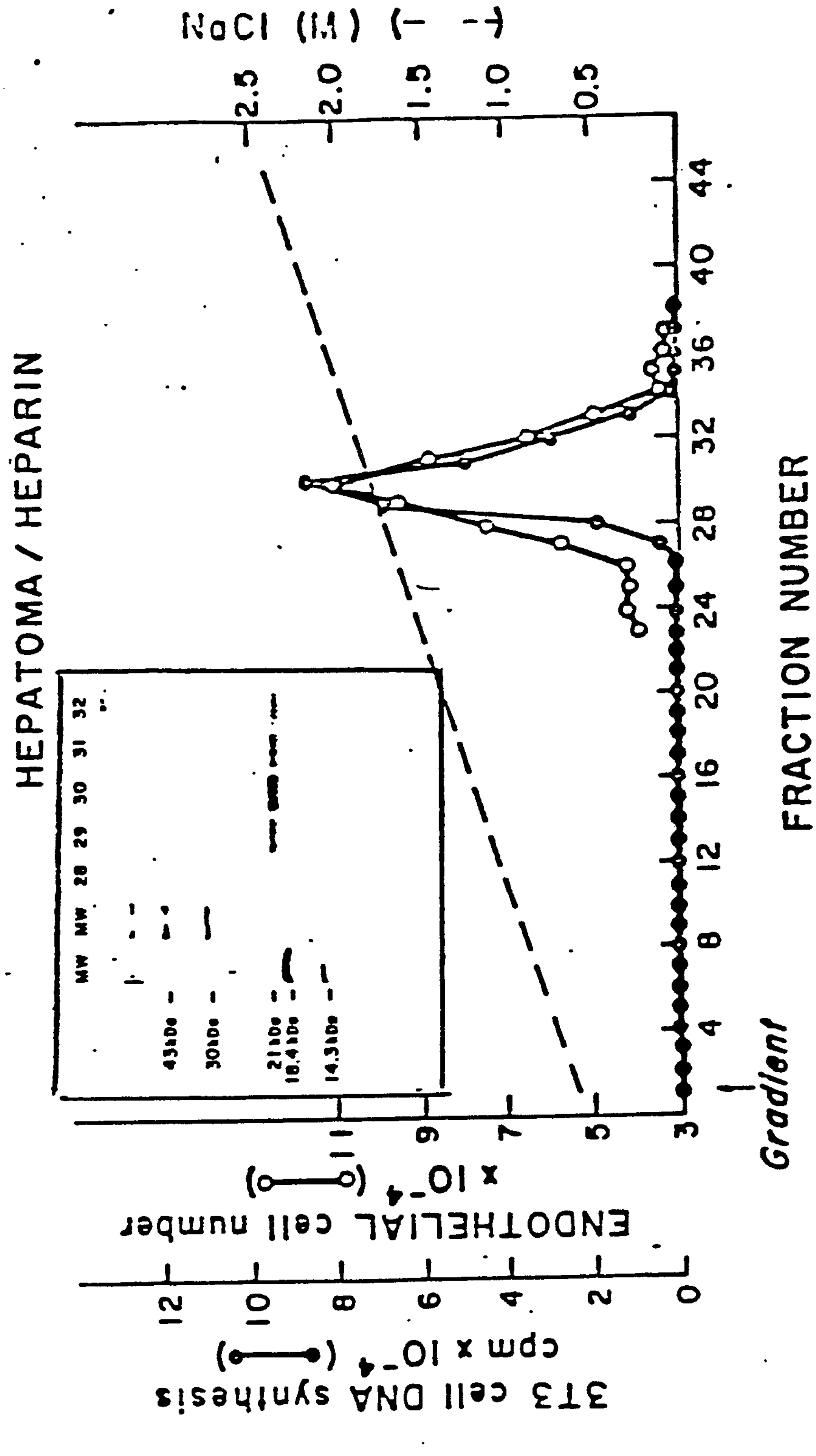

HEPATOMA / HEPARIN
NaCl (M) (– –)
2.5
2.0
1.5
1.0
0.5
FRACTION NUMBER
44
40
36
32
28
24
20
16
12
8
4
Gradient
ENDOTHELIAL cell number x 10^-4 (o—o)
11
9
7
5
3
3T3 cell DNA synthesis cpm x 10^-4 (●—●)
12
10
8
6
4
2
0
MW MW 28 29 30 31 32
43kDa
30kDa
21kDa
18.4kDa
14.3kDa


FIGURE 1

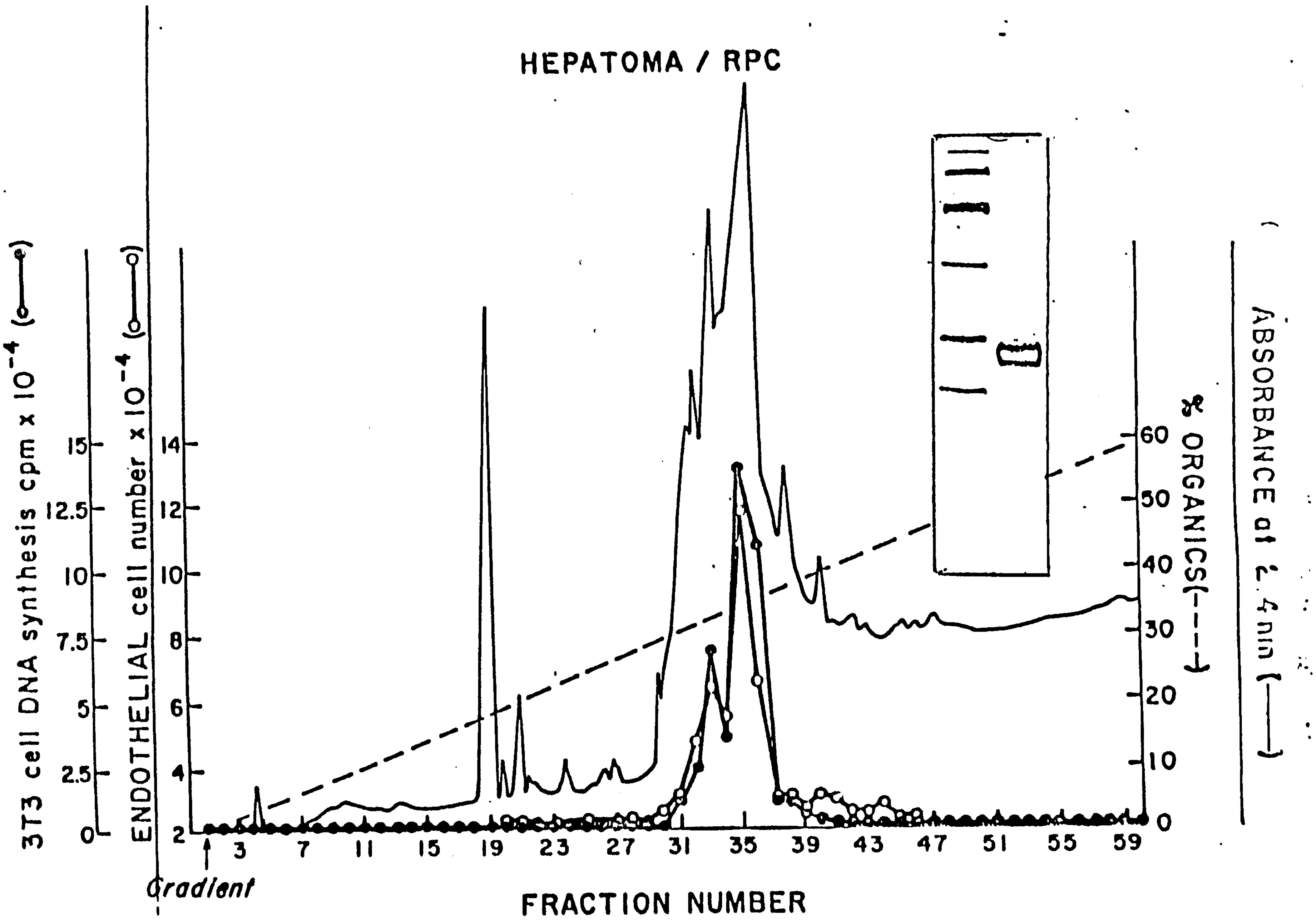
HEPATOMA / RPC
3T3 cell DNA synthesis cpm x 10^-4 (●—●)
ENDOTHELIAL cell number x 10^-4 (○—○)
% ORGANICS(- - - -)
ABSORBANCE at 2.4 nm (——)
0
2.5
5
7.5
10
12.5
15
2
4
6
8
10
12
14
0
10
20
30
40
50
60
Gradient
3
7
11
15
19
23
27
31
35
39
43
47
51
55
59
FRACTION NUMBER

FIGURE 2

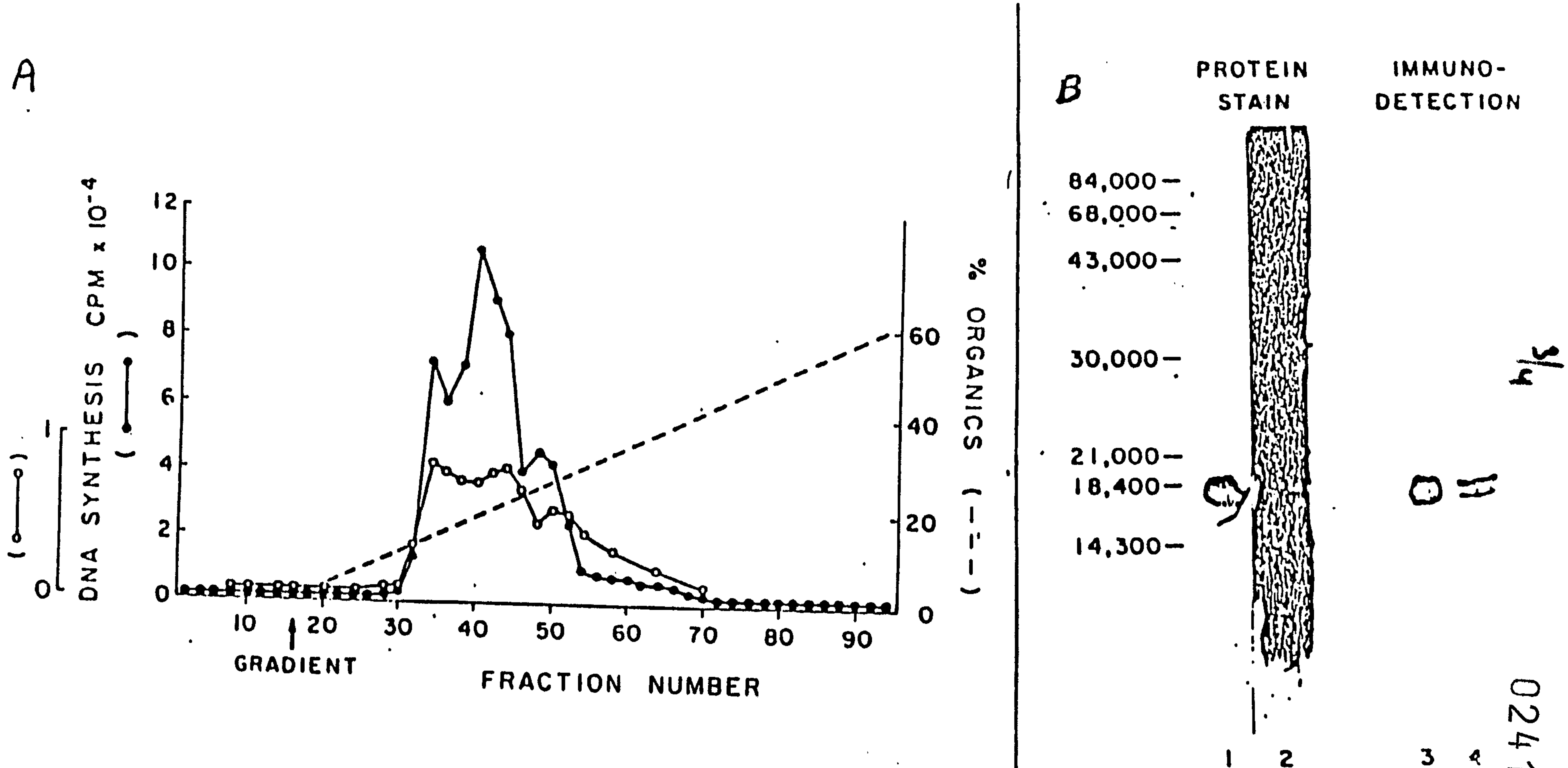
A
DNA SYNTHESIS CPM x 10^-4
0
1
0
2
4
6
8
10
12
10
20
30
40
50
60
70
80
90
GRADIENT
FRACTION NUMBER
% ORGANICS
0
20
40
60
B
PROTEIN STAIN
IMMUNO-DETECTION
84,000—
68,000—
43,000—
30,000—
21,000—
18,400—
14,300—
1
2
3
4

FIGURE 3

| Position | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| Human HDGF peptide fragment | (Thr) | Leu | Pro | Ala | Leu | Pro | Glu | Asp | Gly | Gly | (X) | Gly | Asp | Phe | Pro | Pro | Gly |
| Bovine FGF amino terminus | | | Pro | Ala | Leu | Pro | Glu | Asp | Gly | Gly | Ser | Gly | Asp | Phe | Pro | Pro | Gly |
| Human FGF amino terminus | | | Pro | Ala | Leu | Pro | Glu | Glu | Gly | Gly | Ser | Gly | Asp | Phe | Pro | Pro | Gly |

(X) unidentified amino acid moiety

FIGURE 4